(19) Europäisches Patentamt
European Patent Office
Office européen des brevets

(11) Publication number: **0 209 106**
**B1**

(12) # EUROPEAN PATENT SPECIFICATION

(45) Date of publication of patent specification: **18.10.89**

(51) Int. Cl.⁴: **C 07 D 401/12,** A 61 K 31/44

(21) Application number: **86109650.1**

(22) Date of filing: **14.07.86**

(54) **2-(4-Pyridylaminomethyl)-benzimidazole derivatives and pharmaceutical compositions.**

(30) Priority: **15.07.85 JP 156680/85**

(43) Date of publication of application:
**21.01.87 Bulletin 87/04**

(45) Publication of the grant of the patent:
**18.10.89 Bulletin 89/42**

(84) Designated Contracting States:
**DE FR GB**

(56) References cited:
**US-A-4 506 074**

**CHEMICAL ABSTRACTS, vol. 88, no. 15, April 10, 1978, Columbus, Ohio, USA. RIDA, SAMIA M.; SHAMS EL-DINE, S.A.; LABOUTA, I.M.: "Synthesis of benzimidazole derivatives with potential antihistaminic activity", page 562, column 2, abstract no. 105 224w**

**CHEMICAL ABSTRACTS, vol. 82, no. 7, February 17, 1975, Columbus, Ohio, USA. NAKAO, HIDEO; FUKUSHIMA, MASAMI; SHIMIZU, FUSAAKI; ARAKAWA, MASAO: "Antileukemic agents. 3. Synthesis and antitumor activity of N-(2-chloroethyl)-N-**

**The file contains technical information submitted after the application was filed and not included in this specification**

(73) Proprietor: **Maruishi Seiyaku Kabushiki Kaisha
3-5 Fushimimachi 2-chome
Chuo-ku Osaka (JP)**

(72) Inventor: **Sato, Nobukatsu
1492-2, 3-chome, Rokujyo-nishi-machi
Nara-shi Nara-ken (JP)**
Inventor: **Kuriyama, Haruo
11-25, Shibukawa Terado-cho
Mukou-shi Kyoto-fu (JP)**
Inventor: **Agou, Masanobu
3-2-202, 1-chome Midorida'oka
Ikeda-shi Osaka-fu (JP)**

(74) Representative: **Vossius & Partner
Siebertstrasse 4 P.O. Box 86 07 67
D-8000 München 86 (DE)**

(56) References cited:
**nitrosourea derivatives", page 427, column 2, abstract no. 43 263y**

**Chemical Abstracts Vol 59, Abstract No 3906f**

**Chemical Abstracts Vol 64, Abstract No 2434f**
**NATURE, Vol223, page 785, (1969)**

**EP 0 209 106 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

Picornavirus infections are the most common viral infections in man. This family of viruses consists of the enteroviruses (found primarily in the enteric tract) and the rhinoviruses (found primarily in the nose). The enteroviruses cause a wide range of clinical syndromes ranging from exanthemata, hemorrhagic conjuctivitis and mild upper respiratory infections to more severe diseases such as aseptic meningitis, myocarditis and poliomyelitis. Generally, enterovirus infections of the pediatric population result in greater morbidity and mortality. Recent studies have shown that relatively mild pediatric infections can result in long-term neurological sequelae. Rhinoviruses are responsible for 50% of the common cold infections and cause mild localized infections of the upper respiratory tract.

Since the result of serological testing indicated that there are at least 70 serotypes of rhinoviruses and enteroviruses prophylaxis by vaccination has not been practical or effective except for polioviruses. Therefore, chemotherapy against picornavirus infection deserves serious consideration.

Benzimidazole derivatives are known to show antiviral activity (R. L. Thompson, The Journal of Immunology, Vol. 55 (1947), 345. However, with regard to 2-(N-substituted aminomethyl)-benzimidazoles, only a few studies for antiviral activity were reported. It is known that 2-(anilinomethyl)-benzimidazole is inactive against poliovirus; see K. Otaki et al., Yakugaku Zasshi, Vol. 85 (1965), 926—935.

It is one object of the invention to provide novel 2-(substituted aminomethyl)-benzimidazole derivatives and their acid addition salts having antiviral activity, in particular activity against picornaviruses. It is a further object of the invention to provide pharmaceutical compositions having antiviral activity, in particular antipicornavirus activity.

Thus, this invention relates to 2-(4-pyridylaminomethyl)-benzimidazole derivatives of the general formula (I)

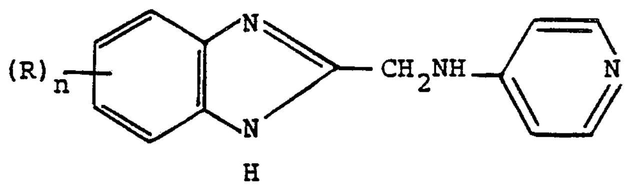

(I)

wherein n is 0, 1 or 2, R is $C_{1-5}$-alkyl, $C_{1-5}$-alkoxy, benzoyl, halogenomethyl, halogen, nitro or amino, their tautomers, and their acid addition salts.

The numbering of the benzimidazole ring system is as follows:

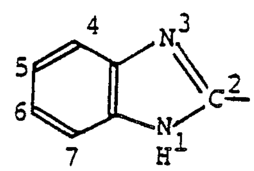

The benzimidazole ring system can exist in a tautomeric form. Consequently, it is permitted to express any substituent located e.g. on the 5-position of the above ring as 5(6).

The compounds of the general formula I form salts with inorganic and organic acids. Typical examples of inorganic acids are hydrochloric acid, hydrobromic acid and sulfuric acid. Typical examples of organic acids are fumaric acid, maleic acid, oxalic acid, succinic acid and malic acid. The alkyl and alkoxy groups are straight or branched groups containing 1 to 5 carbon atoms, preferably 1 to 3 carbon atoms and most preferably 1 or 2 carbon atoms. The term halogen denotes fluoro, chloro, bromo and iodo. The preferred halogen is fluoro and chloro. The preferred number n of substituents on the benzene ring is 0 or 1.

Examples of the preferred compounds of this invention are:

(I) 2-(4-pyridylaminomethyl) benzimidazole dihydrochloride
(II) 2-(4-pyridylaminomethyl)-5(6)-chlorobenzimidazole dihydrochloride
(III) 2-(4-pyridylaminomethyl)-5(6)-benzoylbenzimidzole dihydrochloride
(IV) 2-(4-pyridylaminomethyl)-5(6)-methoxybenzimidazole dihydrochloride
(V) 2-(4-pyridylaminomethyl)-5(6)-methylbenzimidazole dihydrochloride
(VI) 2-(4-pyridylaminomethyl)-5(6)-nitrobenzimidazole dihydrochloride
(VII) 2-(4-pyridylaminomethyl)-5,6-dichlorobenzimidazole dihydrochloride
(VIII) 2-(4-pyridylaminomethyl)-5(6)-aminobenzimidazole trihydrochloride
(IX) 2-(4-pyridylaminomethyl)-5(6)-trifluoromethylbenzimidazole dihydrochloride
(X) 2-(4-pyridylaminomethyl)-5(6)-ethylbenzimidazole dihydrochloride
(XI) 2-(4-pyridylaminomethyl)-4(7)-methylbenzimidazole dihydrochloride
(XII) 2-(4-pyridylaminomethyl)-5,6-dimethylbenzimidazole dihydrochloride

The examples illustrate the preparation of compound of this invention.

Example 1 (Compound I)

(a) Preparation of the starting material (2-chloro-methylbenzimidazole)

It is a known compound and can be obtained by reaction of o-phenylenediamine with monochloroacetic acid.

(b) Preparation of 2-(4-pyridylaminomethyl)-benzimidazole dihydrochloride

A solution of 1.665 g (10 mmol) of 2-chloromethylbenzimidazole and 1.88 g (20 mmol) of 4-aminopyridine in 20 ml of ethanol was refluxed under heating for three hours. The solution was evaporated in vacuo to a dark oil, dissolved in 20 ml of water and washed twice with 20 ml of ethylacetate. The aqueous phase was evaporated in vacuo. The residual oil was dissolved in 3 ml of concentrated hydrochloric acid and chilled (0°C) causing a crystalline precipitate to form. The crystals were filtered with the aid of ethanol. Recrystallization from methanol gave 1.68 g (53.3%) of the title compound (I).

Analysis for $C_{13}H_{12}N_4 \cdot 2HCl \cdot H_2O$

| | | | |
|---|---|---|---|
| Found: | C: 49.93; | H: 5.17; | N: 17.08 |
| Calcd | C: 49.53; | H: 5.12; | N: 17.78 |

Example 2 (Compound IV)

(a) Preparation of 2-chloromethyl-5(6)-methoxybenzimidazole hydrochloride

To 12.5 ml of 3N aqueous hydrochloric acid were added 2.1 g (12 mmol) of 4-methoxy-1,2-phenylenediamine hydrochloride and 1.3 g (17 mmol) of glycolic acid, and the mixture was heated at 120°C for an hour. The reaction solution was cooled to room temperature, and rendered alkaline by the addition of ammonium hydroxide. The solution was filtered under suction, and the precipitate was washed with water and dried to give 2-hydroxymethyl-5(6)-methoxybenzimidazole as a yellow solid. 1 g (5.6 mmol) was added to a stirred solution of 0.75 g (0.46 ml, 6.3 mmol) of thionyl chloride in 10 ml of dry chloroform under cooling with ice. Thereafter the mixture was refluxed for 2 hours. After cooling, to the reaction solution was added dropwise ethanol to decompose the excess thionyl chloride and evaporated to dryness.

The residual solid was triturated with 30 ml of petroleum ether and washed with 20 ml of chloroform to yield 1.24 g (44.3%) of the title compound as reddish violet crystals: mp. 187—191°C

Analysis for $C_9H_9N_2OCl \cdot HCl$

| | | | |
|---|---|---|---|
| Found | C: 46.96; | H: 4.76; | N: 12.07 |
| Calcd | C: 46.37; | H: 4.32; | N: 12.02 |

(b) Preparation of 2-(4-pyridylaminomethyl)-5(6)-methoxybenzimidazole dihydrochloride

A solution of 1.22 g (5.2 mmol) of 2-chloromethyl-5(6)-methoxybenzimidazole hydrochloride in 30 ml of methanol was stirred overnight with 0.53 g of sodium bicarbonate. Then, a crystalline precipitate was filtered off and the filtrate was evaporated in vacuo. The residue and 0.97 g (10.3 mmol) of 4-aminopyridine were dissolved in 10 ml of ethanol and refluxed for 3 hours. After cooling, the reaction solution was filtered and the filtrate was evaporated in vacuo. The residual oil was dissolved in 10 ml of water and washed twice with 20 ml of ethyl acetate. The aqueous phase was evaporated to dryness. The residual oil was dissolved in 1.5 ml of concentrated hydrochloric acid and chilled (0°C) causing a crystalline precipitate to form. The crystals were filtered with the aid of 30 ml of ethanol.

Recrystallization from a mixture of methanol and ethanol gave 0.9 g (48.86%) of the title compound (IV) as white crystals.

Example 3 (Compound VII)

(a) Preparation of 2-chloromethyl-5,6-dichlorobenzimidazole

A solution of 1.77 g (10 mmol) of 4,5-dichloro-1,2-phenylenediamine in 15 ml of 4N aqueous hydrochloric acid was kept at room temperature overnight. To the solution was added 1.42 g (15 mmol) of monochloroacetic acid, and refluxed for 3 hours and filtered. The filtrate was cooled to room temperature, stirred and rendered alkaline by addition of ammonium hydroxide. The separated precipitate was washed with water and dried to give 1.9 g (68%) of the title compound.

(b) Preparation of 2-(4-pyridylaminomethyl)-5,6-dichlorobenzimidazole dihydrochloride

A solution of 1.0 g (4.2 mmol) of 2-chloromethyl-5,6-dichlorobenzimidazole and 1.19 g (12.6 mmol) of 4-aminopyridine in 15 ml of ethanol was refluxed for 5 hours. The reaction solution was cooled to room temperature and evaporated in vacuo. The residual oil was crystallized from acetone. The precipitate was dissolved in a small amount of methanol, 2 ml of concentrated hydrochloric acid were added and the mixture chilled (0°C) causing a crystalline precipitate to form. This precipitate was filtered and dried to give 0.55 g (35.77%) as creamy crystals.

Analysis for $C_{13}H_{10}N_4Cl_2 \cdot 2HCl$

| | | | |
|---|---|---|---|
| Found | C: 42.17; | H: 3.29; | N: 15.12 |
| Calcd | C: 42.65; | H: 3.30; | N: 15.31 |

Example 4 (Compound IX)

(a) Preparation of 4-trifluoromethyl-1,2-phenylene diamine

A solution of 6.18 g (30 mmol) of 4-amino-3-nitrobenzotrifluoride in 150 ml of ethanol was hydrogenated under stirring at atmospheric pressure in the presence of 1.5 g of 5% Pd/C. The catalyst was filtered off. The filtrate was evaporated and the residue was crystallized from petroleum ether to give 4.5 g (85.17%) of the title compound as white crystals.

(b) Preparation of 2-chloromethyl-5(6)-trifluoromethylbenzimidazole

To a solution of 3 g (17.0 mmol) of 4-trifluoromethyl-1,2-phenylenediamine in 17 g (68.1 mmol) of 4N aqueous hydrochloric acid was added 2.4 g (25.5 mmol) of monochloroacetic acid and refluxed for 2 hours. The reaction solution was cooled to room temperature and rendered alkaline by addition of ammonium hydroxide. The separated precipitate was washed with water and dried to give 2.7 g (67.56%) of the title compound.

(c) Preparation of 2-(4-pyridylaminomethyl)-5(6)-trifluoromethylbenzimidazole dihydrochloride

A solution of 2 g (9 mmol) of 2-chloromethyl-5(6)-trifluoromethylbenzimidazole, 1.7 g (18 mmol) of 4-aminopyridine and 1.8 g (18 mmol) of triethylamine in 30 ml of ethanol was refluxed for 5 hours. The reaction solution was evaporated in vacuo, dissolved in 20 ml of water, and the pH was adjusted to 5—7 with concentrated hydrochloric acid. The solution was washed twice with 20 ml of ethyl acetate.

The aqueous phase was evaporated in vacuo. To the residue was added 2.25 g (22.5 mmol) of concentrated hydrochloric acid and the mixture evaporated in vacuo to dryness. The residue was crystallized from ethanol to give 1.05 g (31.99%) of the title compound as white crystals.

Example 5 (Compound X)

(a) Preparation of 4-ethyl-2-nitroaniline

According to the method of synthesis for 2-amino-3-nitrotoluene (Organic Syntheses Collective Volume IV, p. 42—45) 6 g of 4-ethylaniline was added dropwise to 30 ml of acetic anhydride and then the mixture was treated dropwise with 6.3 ml (100 mmol) of 70% nitric acid at 12—13°C. Thereafter the mixture was stirred for some time at 10—12°C, the reaction solution was poured into ice water. Oily product was separated and heated with 15 ml of concentrated hydrochloric for one hour. The reaction solution was alkalified with dilute aqueous sodium hydroxide and then partitioned with 100 ml of chloroform. The organic layer was washed with water and dried on anhydrous sodium sulfate. Removal of the solvent in vacuo gave 5.76 g (69.32%) of 4-ethyl-2-nitroaniline.

(b) Preparation of 4-ethyl-1,2-phenylenediamine

A solution of 5.7 g (34.3 mmol) of 4-ethyl-2-nitroaniline in 150 ml of ethanol was hydrogenated under stirring at atmospheric pressure in the presence of 1.8 g of 5% Pd/C.

The catalyst was filtered off, and removal of the solvent in vacuo gave an oil (4 g). Then, 6 g (58.8 mmol) of concentrated hydrochloric acid was added to the solution, that gave 5.4 g (75.33%) of 4-ethyl-1,2-phenylenediamine dihydrochloride.

(c) Preparation of 2-chloromethyl-5(6)-ethylbenzimidazole

To a solution of 2 g (9.6 mmol) of 4-ethyl-1,2-phenylenediamine dihydrochloride in 10 ml of 4N aqueous hydrochloric acid was added 1.36 g (14.4 mmol) of monochloroacetic and refluxed for 3 hours. After cooling, the reaction solution was slightly alkalified with ammonium hydroxide. After filtration of the solid, it was washed with water to yield 1.8 g (96.77%) of 2-chloromethyl-5(6)-ethylbenzimidazole.

(d) Preparation of 2-(4-pyridylaminomethyl)-5(6)-ethylbenzimidazole

To a solution of 1.5 g (7.7 mmol) of 2-chloromethyl-5(6)-ethylbenzimidazole in 30 ml of ethanol was added 1.45 g (15.4 mmol) of 4-aminopyridine and refluxed for 2 hours. Thereafter the reaction mixture was evaporated in vacuo, the residue was dissolved in 20 ml of water and washed twice with 20 ml of ethyl acetate. The aqueous phase was evaporated in vacuo. Then, the residue was dissolved in 2.1 g (21 mmol) of concentrated hydrochloric acid, evaporated to dryness in vacuo and crystallized from ethanol to give 0.8 g (31.94%) of 2-(4-pyridylaminomethyl)-5(6)-ethylbenzimidazole dihydrochloride as white crystals.

Analysis for $C_{15}H_{16}N_4 \cdot 2HCl \cdot H_2O$

| | | | |
|---|---|---|---|
| Found | C: 52.09; | H: 5.82; | N: 16.06 |
| Calcd | C: 52.44; | H: 5.54; | N: 16.32 |

Example 6 (Compound XI)

(a) Preparation of 3-methyl-1,2-phenylenediamine

A solution of 4.56 (30 mmol) of 2-methyl-6-nitroaniline in 150 ml of methanol was hydrogenated under stirring at atmospheric pressure in the presence of 0.9 g of 5% Pd/C. The catalyst was filtered off. The solvent was evaporated, and the residue was crystallized from petroleum ether to give 3.25 g (88.67%) of the title compound as gray crystals.

(b) Preparation of 2-chloromethyl-4-methylbenzimidazole

A solution of 1.83 g (15 mmol) of 3-methyl-1,2-phenylenediamine in 20 ml of 4N aqueous hydrochloric acid was kept at room temperature overnight. To the solution was added 2.13 g (22.5 mmol) of monochloroacetic acid and refluxed for 3 hours. The reaction solution was cooled to room temperature, rendered alkaline by addition of ammonium hydroxide and extracted with ethyl acetate. The ethyl acetate

layer was washed with water and dried over anhydrous sodium sulfate. The solvent was evaporated in vacuo and the residue was crystallized from ethyl ether to give 0.8 g (29.52%) of the title compound.

(c) Preparation of 2-(4-pyridylaminomethyl)-4(7)-methylbenzimidazole dihydrochloride

A solution of 0.7 g (3.88 mmol) of 2-chloro-4-methylbenzimidazole and 0.73 g (7.75 mmol) of 4-aminopyridine in 10 ml of ethanol was refluxed for 3 hours. The reaction solution was cooled to room temperature and filtered. Thereafter the filtrate was evaporated in vacuo, the residue was dissolved in water and washed several times with ethyl acetate. The aqueous phase was evaporated in vacuo. To the residual oil was added 2 ml of concentrated hydrochloric acid and the mixture dried in vacuo. The residue was crystallized from ethanol to give 0.25 g (27.47%) of the title compound.

Analysis for $C_{14}H_{14}N_4 \cdot 2HCl \cdot H_2O$

| | | | |
|---|---|---|---|
| Found | C: 51.99; | H: 5.42; | N: 17.19 |
| Calcd | C: 51.09; | H: 5.51; | N: 17.02 |

The physical properties of the representative compounds of this invention will be listed below:

Compound I

Formula: $C_{13}H_{12}N_4 \cdot 2HCl \cdot H_2O$
Melting point: 195—197°C
IR $\nu_{max}$ $cm^{-1}$: 1650, 1540, 1190, 750, 650
$^1H$-NMR ($D_2O$, DMSO-$d_6$) δ: 6.0 (2H), 7.0 (2H), 7.4—7.9 (4H), 8.4 (2H).

Compound II

Formula: $C_{13}H_{11}N_4Cl \cdot 2HCl \cdot H_2O$
Melting point: 262—265°C
IR $\nu_{max}$ $cm^{-1}$: 1660, 1540, 1190
$^1$NMR ($D_2O$, DMSO-$d_6$) δ: 5.9 (2H), 7.0 (2H), 7.3—7.9 (3H), 8.4 (2H).

Compound III

Formula: $C_{20}H_{16}N_4 \cdot 2HCl \cdot H_2O$
Melting point: 163—168°C
IR $\nu_{max}$ $cm^{-1}$: 1660, 1640, 1530, 1280, 1180, 840, 700
$^1H$-NMR ($D_2O$, DMSO-$d_6$) δ: 5.8 (2H), 7.0 (2H), 7.5—8.0 (8H), 8.4 (2H).

Compound IV

Formula: $C_{14}H_{14}N_4O \cdot 2HCl \cdot 1.5H_2O$
Melting point: 196—199°C
IR $\nu_{max}$ $cm^{-1}$: 1660, 1540, 1200, 840
$^1H$-NMR ($D_2O$, DMSO-$d_6$) δ: 3.9 (3H), 6.0 (2H), 7.0 (2H), 7.2 (2H), 7.7 (1H), 8.4 (2H).

Compound V

Formula: $C_{14}H_{14}N_4 \cdot 2HCl \cdot H_2O$
Melting point: 199—203°C
IR $\nu_{max}$ $cm^{-1}$: 1650, 1540, 1200, 830
$^1H$-NMR ($D_2O$, DMSO-$d_6$) δ: 2.5 (3H), 6.0 (2H), 7.0 (2H), 7.2—7.8 (3H), 8.4 (2H).

Compound VI

Formula: $C_{13}H_{13}N_5O_2 \cdot 2HCl$
Melting point: >300°C
IR $\nu_{max}$ $cm^{-1}$: 1650, 1520, 1340, 1210, 830
$^1H$-NMR ($D_2O$, DMSO-$d_6$) δ: 5.9 (2H), 7.0 (2H), 7.7—8.6 (3H).

Compound VII

Formula: $C_{13}H_{10}N_4Cl_2 \cdot 2HCl$
Melting point: >300°C
IR $\nu_{max}$ $cm^{-1}$: 1650, 1540, 1190, 840
$^1H$-NMR ($D_2O$, DMSO-$d_6$) δ: 5.8 (2H), 7.0 (2H), 7.9 (2H), 8.3 (H).

Compound VIII

Formula: $C_{13}H_{13}N_5 \cdot 3HCl$
Melting point: 217—222°C
IR $\nu_{max}$ $cm^{-1}$: 3360, 3160, 1660, 850, 600
$^1H$-NMR ($D_2O$, DMSO-$d_6$) δ: 5.9 (2H), 7.0 (2H), 7.4—7.6 (1H), 7.8—8.0 (2H), 8.2 (2H).

Compound IX

Formula: $C_{14}H_{11}N_4F_3 \cdot 2HCl$

Melting point: 154—156°C
IR $\nu_{max}$ $cm^{-1}$: 1660, 1330, 1200, 1130, 820
$^1H$-NMR ($D_2O$, DMSO-$d_6$) δ: 5.9 (2H), 7.0 (2H), 7.6—8.1 (3H), 8.3 (2H).

Compound X
Formula: $C_{15}H_{16}N_4 \cdot 2HCl \cdot H_2O$
Melting point: 185—188°C
IR $\nu_{max}$ $cm^{-1}$: 1650, 1540, 1200, 830
$^1H$-NMR ($D_2O$, DMSO-$d_6$) δ: 1.2 (3H), 2.8 (2H), 6.0 (2H), 7.0 (2H), 7.3—7.9 (3H), 8.3 (2H).

Compound XI
Formula: $C_{14}H_{14}N_4 \cdot 2HCl \cdot H_2O$
Melting point: 230—237°C
IR $\nu_{max}$ $cm^{-1}$: 1640, 1530, 1180, 840, 780, 740
$^1H$-NMR ($D_2O$, DMSO-$d_6$) δ: 2.6 (3H), 6.0 (2H), 7.0 (2H), 7.1—7.7 (3H), 8.5 (2H).

Compound XII
Formula: $C_{15}H_{16}N_4 \cdot 2HCl$
Melting point: 295—298°C
IR $\nu_{max}$ $cm^{-1}$: 1650, 1540, 1180, 840
$^1H$-NMR ($D_2O$, DMSO-$d_6$) δ: 2.3 (6H), 5.9 (2H), 6.9 (2H), 7.5 (2H), 8.4 (2H).

Test results of antiviral activity studies with compounds of the invention are described in the following experiments 1 and 2 (in vitro antiviral activity).

Experiment 1

Inhibition of cytopathic effects

For studying the cytopathic effect (CPE), LLC-$MK_2$ cells were seeded in microtest plates at a concentration of $2.0 \times 10^4$ cells per well in 0.1 ml of Eagle's minimum essential medium (MEM) containing 5% fetal calf serum and 50 μg of kanamycin per ml. After 24b of growth at 37°C in a $CO_2$ (5% $CO_2$, 95% air) incubator, the cultures were 80% monolayered and ready for use. Cell cultures in the microtest plates were drained and (except compound toxicity and cell controls) were challenged with 20 μl (100—300 $TCID_{50}$) of enterovirus type 70. The cultures are incubated for 1 h at 33°C. The virus inoculum was removed and cells were then cultured with MEM containing the compounds to be tested serially diluted. The cell cultures were maintained at 33°C in a $CO_2$ incubator and examined microscopically at 48, 72, 96 and 120 h after challenge for compound cytotoxicity and virus CPE.

The test results of Experiment 1 are shown in Table 1. The lowest concentration of a compound that reduced virus CPE and caused morphological cytotoxicity by 50% or more was considered to be the $IC_{50}$ and $CD_{50}$, respectively. The chemotherapeutic index (CI) was calculated by the following equation: $CI = CD_{50}/IC_{50}$.

Experiment 2

In plaque reduction assays, compounds I to XII selectively inhibited the plaque formation by human enteroviruses, such as polioviruses (type 1, 2 and 3), echoviruses (type 6, 11 and 25), coxsackieviruses (type A9, A16, B2, B3 and B4), and enterovirus 71.

TABLE 1

| Compound No. | $IC_{50}$ (µg/ml) | $CD_{50}$ (µg/ml) | CI |
|---|---|---|---|
| I | 3.7 | 562.3 | 152 |
| II | 1.8 | 177.8 | 99 |
| III | >100.0 | >100.0 | |
| IV | 27.6 | 1767.8 | 64 |
| V | 1.8 | 562.3 | 311 |
| VI | >100.0 | >100.0 | |
| VII | 1.4 | 176.8 | 126 |
| VIII | >100.0 | >100.0 | |
| IX | 5.5 | 353.6 | 64 |
| X | 0.98 | 353.6 | 362 |
| XI | >100.0 | >100.0 | |
| XII | 3.0 | 77.1 | 239 |

The compounds of the general formula I may be formulated into antiviral pharmaceutical compositions for oral or parenteral administration.

## Claims

1. 2-(4-pyridylaminomethyl)-benzimidazole derivatives of the general formula I

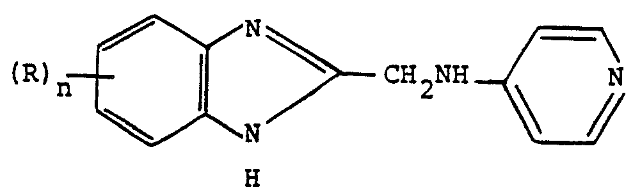

(I)

wherein n is 0, 1 or 2, R is $C_{1-5}$-alkyl, $C_{1-5}$-alkoxy, benzoyl, halogenomethyl, halogen, nitro or amino, their tautomers, and their acid addition salts.

2. Pharmaceutical composition, comprising a compound according to claim 1.

3. Pharmaceutical composition having antiviral activity, comprising a compound according to claim 1.

## Patentansprüche

1. 2-(4-Pyridylaminomethyl)-benzimidazol-Derivate der allgemeinen Formel I

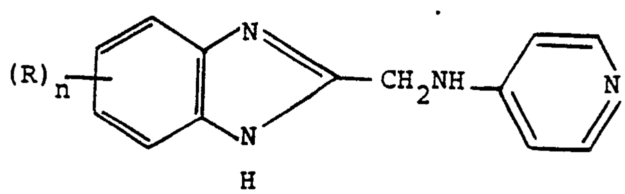

(I)

in der n 0, 1 oder 2 ist, R eine $C_{1-5}$-Alkyl-, $C_{1-5}$-Alkoxy-, Benzoyl-, Halogenmethyl-, Halogen-, Nitro- oder Aminogruppe bedeutet, ihre Tautomeren und ihre Säureadditionssalze.

2. Arzneimittel, umfassend eine Verbindung nach Anspruch 1.
3. Arzneimittel mit antiviraler Wirksamkeit, umfassend eine Verbindung nach Anspruch 1.

**Revendications**

1. Dérivés du 2-(4-pyridylaminométhyl)-benzimidazole, de la formule générale I:

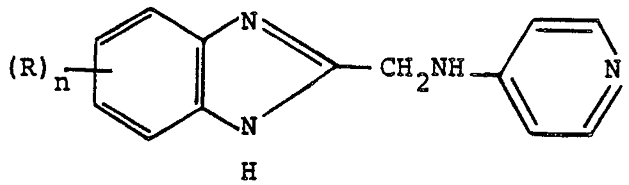

(I)

dans laquelle n est 0, 1 ou 2, R est un groupe alkyle en $C_{1-5}$, un groupe alkoxy en $C_{1-5}$, un groupe benzoyle, halogénométhyle, halogène, nitro ou amino, leurs tautomères et leurs sels d'addition acides.
2. Composition pharmaceutique, comprenant un composé suivant la revendication 1.
3. Composition pharmaceutique ayant une activité antivirale, comprenant un composé suivant la revendication 1.